# EUROPEAN PATENT SPECIFICATION

(11) **EP 4 294 223 B1**
(45) Date of publication and mention of the grant of the patent: **02.04.2025**
(21) Application number: 22707067.9
(22) Date of filing: 16.02.2022
(51) Int. Cl.: A24F 42/00, A61M 15/06

(54) **HOLDER WITH DOUBLE ACTIVATION PREVENTION**
HALTER MIT VERHINDERUNG EINER DOPPELTEN AKTIVIERUNG
SUPPORT AVEC PRÉVENTION DE DOUBLE ACTIVATION

(30) Priority: 17.02.2021 EP 21157709
(43) Date of publication of application: 27.12.2023
(73) Proprietor: Philip Morris Products S.A., 2000 Neuchâtel (CH)
(72) Inventor: BLÄTTLER, Olivier, 3097 Liebefeld-Berne (CH); CROOK, Benjamin Paul, 3097 Liebefeld-Berne (CH); DAYIOGLU, Onur, 2000 Neuchâtel (CH); LOSER, Peter, 3097 Liebefeld-Berne (CH); MEYLAN, Grégoire Pierre Adolphe, 3097 Liebefeld-Berne (CH); STOHR, Dominique Paul Gabriel, 3097 Liebefeld-Berne (CH)
(74) Representative: Reddie & Grose LLP
(86) International application number: PCT/EP2022/053754
(87) International publication number: WO 2022/175302

(56) References cited:
- WO-A1-2019/195944
- WO-A1-2020/178714

## Description

The present invention relates to a holder for an inhaler article. In particular, this invention relates to a holder for an inhaler article, the holder comprising a way to prevent double activation of an inhaler article.

Certain inhaler articles retain capsules containing dry powder. These capsules may be activated by piercing the capsule wall. Typically, the user does not receive an indication that the capsule has been pierced or activated. This may lead to the user activating the capsule multiple times. Activating the capsule more than once may alter the powder delivery by increasing the size of a pierced hole or increasing the number of holes resulting in increased powder delivery and a shortened user experience.

WO 2020/178714 A1 describes a holder for an inhaler article comprising a housing defining an inhaler article cavity. A piercing element is fixed to and extends into the inhaler article cavity. The holder also comprises: a sleeve disposed within the inhaler article cavity and configured to retain an inhaler article, the sleeve being movable along the housing longitudinal axis; and a spring element configured to bias the sleeve toward the open proximal end of the housing.

It would be desirable to provide a holder for an inhaler article that may prevent users from accidentally piercing a capsule or activating an inhaler article more than once when the inhaler article is inserted into the holder for use. Preventing users from accidentally piercing a capsule or activating an inhaler article more than once may help to increase the consistency of performance from one inhaler article to another, for example by helping to increase the consistency of powder delivery.

It would be desirable to provide a holder for an inhaler article that prevents multiple activations of a powder capsule of an inhaler article received within the holder. It would also be desirable to provide an inhaler system that is convenient to use by a consumer. It would also be desirable to provide an inhaler system which is simple to construct and use and which does not require electronic circuitry to prevent double activation of an inhaler article of the inhaler system.

The present disclosure relates to a holder for an inhaler article. The holder may comprise a housing. The housing may comprise a housing cavity. The housing cavity may be for receiving an inhaler article. The holder may further comprise an engagement member located at least partially within the housing cavity. The engagement member may be movable relative to the housing between an upstream position and a downstream position. The engagement member may comprise a first follower member. The housing may comprise a first guide. The housing may comprise a first latch. The first follower member may be arranged to follow the first guide between a first position, a second position, and a third position. The first latch may be arranged such that the first follower member may pass from the first position to the second position, but may not return to the first position without first passing the third position.

According to the present invention, there is provided a holder for an inhaler article. The holder comprises a housing comprising a housing cavity. The holder further comprises an engagement member located at least partially within the housing cavity and being movable relative to the housing between an upstream position and a downstream position. The engagement member comprises a first follower member. The housing comprises a first guide and a first latch, the first follower member being arranged to follow the first guide between a first position, a second position, and a third position, the first latch being arranged such that the first follower member may pass from the first position to the second position, but may not return to the first position without first passing the third position.

The provision of the arrangement of the present invention may advantageously prevent double activation of an inhaler article inserted into the holder for use. In particular, when an inhaler article is inserted into the holder, specifically into the housing cavity of the housing of the holder, the engagement member may be pushed from the downstream position to the upstream position. This action may activate an inhaler article received in the housing cavity. This action may also advance the first follower member along the first guide from the first position to the second position, passing the first latch. Once the inhaler article is activated, the engagement member may move from the upstream position to the downstream position. This action may advance the first follower member from the second position to the third position, the latch preventing movement from the second position directly back to the first position. In this way, the holder may prevent the first follower member from returning to the second position, thereby preventing further activation of the inhaler article.

The first follower member may pass from the first position to the second position without passing the third position.

The engagement member may be configured to move relative to the housing along the longitudinal axis of the holder.

The engagement member may engage with the housing of the holder. For example, the engagement member may engage with the first guide of the housing. The engagement member may comprise a body, the body being sized to be received in the housing cavity. The engagement member may comprise a protrusion on the body. The protrusion may form the first follower member.

The first follower member, first guide, and first latch as set out above may together form a first double activation prevention mechanism. The first double activation prevention mechanism may further comprise additional features as set out below.

The housing may be formed of any rigid material. The housing may be formed of a polymeric material. Polymeric materials useful for forming the housing include polycarbonate, polypropylene, polyethylene, nylon, acrylonitrile butadiene styrene, styrene acrylonitrile, polyacrylate, polystyrene, PBT polyester, PET polyester, polyoxymethylene, polysulfone, polyethersulfone, polyethereetherketone, or liquid crystal polymer, for example. Polyproplyene, polyethylene or co-polymers thereof are preferred materials for forming the housing.

As used herein with reference to the invention, the terms "upstream" and "downstream", are used to describe the relative positions of components, or portions of components, of the inhaler system, holder, or inhaler article in relation to the direction in which air flows through the inhaler article during use thereof. Components, or portions of components, of the inhaler system, holder, or inhaler article may be described as being upstream or downstream of one another based on their relative positions between an upstream end of the inhaler system and a downstream end of the inhaler system.

As used herein with reference to the invention, the term "longitudinal axis of the holder" refers to the axis extending between the upstream end of the holder and the downstream end of the holder. A longitudinal direction is a direction parallel to the longitudinal axis of the holder.

The holder may further comprise a sleeve comprising a sleeve cavity for receiving an inhaler article, the sleeve being located at least partially within the housing cavity movable relative to the housing between an upstream position and a downstream position, the upstream and downstream positions corresponding to the upstream and downstream positions of the engagement member.

The sleeve may be moveable relative to the housing along the longitudinal axis of the holder.

The provision of a sleeve may advantageously provide a convenient way to receive and secure an inhaler article in the holder. The sleeve may have any shape. The sleeve may have an outer diameter substantially the same as or slightly lower than the internal diameter of the housing cavity. The sleeve cavity may have an internal diameter substantially the same or slightly larger than the outer diameter of an inhaler article for use with the holder. The sleeve may be substantially cylindrical in shape.

The sleeve may comprise at least one air inlet. The at least one air inlet may allow air to enter the sleeve cavity at a location upstream of the downstream end of the sleeve. The at least one air inlet may comprise a plurality of openings circumscribing the sleeve. The provision of at least one air inlet may advantageously allow air to enter the sleeve cavity, pass upstream to the upstream end of an inhaler article received in the sleeve cavity, and enter the inhaler article.

The engagement member and the sleeve may be sized and positioned in the housing cavity such that the movement of the sleeve in an upstream direction acts to urge the engagement member in an upstream direction, and the movement of the engagement member in a downstream direction acts to urge the engagement member in a downstream direction.

In use, when an inhaler article is inserted into the sleeve, the sleeve is pushed and moved in an upstream direction. This movement also pushes the engagement member in an upstream direction. Similarly, when the engagement member moves in a downstream direction, the engagement member may push the sleeve in a downstream direction.

The engagement member and the sleeve are both disposed at least partially within the housing cavity and are able to move upstream and downstream relative to the housing. The engagement member may be disposed upstream of the sleeve in the housing cavity. The engagement member may be sized such that it cannot pass the sleeve in the longitudinal direction of the holder. The engagement member may have an outer diameter which is substantially the same as or slightly lower than the internal diameter of the housing cavity. This may advantageously ensure that the sleeve is not able to pass the engagement member when the sleeve moves in an upstream direction and ensures that the engagement member is pushed in an upstream direction by the sleeve.

In addition to being moveable in an upstream and downstream direction along the axis of the holder, the engagement member may be rotatable about the axis of the holder relative to the housing.

As set out in more detail below, the provision that the engagement member may be rotatable about an axis of the holder may allow the double activation mechanism to be reset.

The engagement member may be rotatable through 360 degrees about the axis of the holder. The engagement member may be rotatable through an angle less than 360 degrees about the axis of the holder. For example, the engagement member may be rotatable through less than 180 degrees, less than 90 degrees, less than 45 degrees, or less than 30 degrees. The engagement member may be rotatable through about 20 degrees.

The engagement member may be rotatable about an axis of the holder relative to the sleeve.

The engagement member may be connected to the sleeve in such a way as to allow the rotation of the engagement member relative to the sleeve. Alternatively, the engagement member may not be connected to the sleeve to allow rotation of the engagement member relative to the sleeve. Where the engagement member is not connected to the sleeve, the sleeve may abut and push the engagement member when the sleeve is moved in an upstream direction.

The first guide may be configured such that, once the first follower member is in the third position, it is unable to return to the second position.

This provision may advantageously further help to prevent double activation of the inhaler article since the first follower member is unable to return to the second position.

Once the first follower member is in the third position, it may be unable to return directly to the second position. In other words, the first follower member may be unable to return from the third position to the second position by following the same route taken when moving from the second position to the third position.

The first guide may be configured to allow the first follower member to move from the third position back to the second position by a different route than the route the first follower member takes when moving from the second position to the third position.

The first guide may be configured to only allow the first follower member to move from the third position to the second position by passing through the first position.

The first guide may comprise a ledge on which the first follower member rests when it is in the third position to prevent the first follower member from moving from the third position to the second position.

The first guide may comprise a track in the inner surface of the housing.

The track may comprise a plurality of projections which project inwardly from the inner surface of the housing configured to engage with the first follower member. The track may comprise a groove, slot, or other recess formed in the inner surface of the housing, the groove, slot, or other recess configured to engage with the first follower member. Where the track comprises groove, slot, or other recess, the groove, slot, or other recess may extend through the housing to form an opening. This may advantageously allow a user to see the position of the first follower member in the track from the outside of the holder.

The movement of the engagement member from the downstream position to the upstream position may cause the first follower member to move from the first position to the second position along the first guide.

In other words, the movement of the engagement member may be linked to the movement of the first follower member. Furthermore, the engagement member, the first follower member, and the first guide may be configured such that the first follower member may be in the first position when the engagement member is in the downstream position, the first follower member may be in the second position when the engagement member is in the upstream position, and movement of the engagement member from the downstream position to the upstream position may move the first follower member from the first position to the second position.

The second position of the first follower member in the first guide may be located upstream of the first position of the first follower member in the first guide.

In use, when a user inserts an inhaler article into the housing cavity of the housing, the engagement member may be moved from the downstream position to the upstream position. The movement of the engagement member from the downstream position to the upstream position may cause the first follower member to move from the first position to the second position.

The movement of the engagement member from the upstream position to the downstream position may cause the first follower member to move from the second position to the third position.

Once the engagement member has moved the first follower member from the first position to the second position, a subsequent movement of the engagement member from the upstream position to the downstream position may cause the first follower member to move from the second position to the third position.

The third position of the first follower member in the first guide may be located downstream of the second position of the first follower member in the first guide.

In use, when the engagement member is moved from the upstream position to the downstream position, the first follower member may move from the second position to the third position. The engagement member is prevented from moving from the second position to the first position by the latch.

The first latch may be any latch. The first latch may intersect the first guide between the first position and the second position. The first latch may be configured to deflect or otherwise move as the first follower member moves from the first position to the second position allowing the first follower member to pass from the first position to the second position. The first latch may be configured not to deflect or otherwise move as the first follower member moves from the second position to the third position, thereby preventing the first follower member from passing back to the first position without first passing the third position.

The first latch may comprise a portion of resilient material. The first latch may be attached at one end to the housing.

The first latch may comprise a leaf spring. The leaf spring may be attached at one end to the housing and may extend from there across the first guide between the first position and the second position.

The provision of a latch comprising a leaf spring may advantageously provide a convenient way to allow movement of the first follower member between the first position and second position in only one direction with the need for few moving parts.

The first latch may comprise an inflexible portion and a resiliently flexible portion, the resiliently flexible portion acting to urge the inflexible portion into a position between the first and second positions of the first follower member.

The first latch may be pivotally coupled to the housing.

In use, the first follower member pushes the inflexible portion of the first latch as the first follower member passes from the first position to the second position, the first latch pivoting out of the way to allow the first follower member to pass. Once the first follower member has reached the second position, the resiliently flexible portion urges the inflexible portion into a position between the first and second positions of the first follower member. The resiliently flexible portion may act on a fixed portion of the housing.

The inflexible portion and the resiliently flexible portion of the first latch may be formed from different materials. The inflexible portion and the resiliently flexible portion may be formed from the same material. The inflexible portion and the resiliently flexible portion may be integrally formed. For example, the first latch may be formed from a single piece of polymeric material, the inflexible potion having a greater thickness, and therefore a greater stiffness, than the resiliently flexible portion which has a lower thickness, and lower stiffness.

The provision of a first latch comprising an inflexible portion and a resiliently flexible portion may improve the wear resistance and life span of the first latch since the first follower member will come into contact with the inflexible portion, which may have a high wear resistance, and not the resiliently flexible portion, which may have a lower wear resistance.

The housing may comprise a first stop between the third position and first position of the first follower member arranged to prevent the first follower member from moving from the third position to the first position.

By preventing the first follower member from moving from the third position to the first position, the provision of the stop may advantageously prevent the first follower member from moving from the third position to the second position once the first follower member has passed through the second position once. This may further prevent a second movement of the engagement member from the downstream position to the upstream position thereby preventing double activation of an inhaler article.

The first stop may be fixed in position relative to the first guide thereby permanently preventing movement of the first follower member from the third position to the first position.

The first stop may be movable between a closed position in which the first stop prevents the first follower from moving from the third position to the first position, and an open position in which the first follower member is able to move from the third position to the first position.

The provision of a first stop movable between a closed and an open position may advantageously allow a user to reset the holder, and specifically to reset the double activation prevention mechanism. In use, while the provision of the engagement member comprising the first follower member, the latch, and the first guide may prevent a user from inadvertently activating an inhaler article for a second time by pushing the inhaler article from a downstream position to an upstream position, the provision of a movable first stop may allow a user to choose when to allow the first follower member to move directly from the third position to the first position. From the first position, first follower member may move to the second position allowing the engagement member to move from the downstream position to the upstream position, thereby activating an inhaler article for a second time. The user may move the first stop from the closed position to the open position to reset the holder when the user inserts a new inhaler article. In this way, the holder may be used repeatedly with several consecutive inhaler articles.

The holder may further comprise an engagement member biasing element configured to urge the first follower member from the third position to the first position.

The provision of a biasing element may mean that the first follower member moves from the third position to the first position when the first stop is moved from the closed to the open position. The provision of the biasing element may allow the holder to reset without a user needing to physically move the first follower member from the third position to the first position. The provision of the biasing element may also advantageously allow the holder to be reset when the holder is in any orientation. The provision of a biasing element may also advantageously ensure the first follower member remains on the ledge when in the third position to prevent the first follower member returning to the second position.

In use, when the user moves the first stop from the closed position to the open position, the biasing element may cause the engagement member to move. This movement may cause the first follower member to move from the third position to the first position.

The engagement member biasing element may act to rotate the engagement member about the longitudinal axis of the holder in a direction which urges the first follower member from the third position to the first position.

The engagement member biasing element may be a spring configured to apply a torsional force to the engagement member.

The spring may advantageously provide a convenient way to bias the engagement member.

The spring may act between a portion of the engagement member and a portion of the housing. The spring may comprise one or more of a coil spring, a helical spring, or a spiral spring. At least a portion of the spring may be disposed within the engagement member.

The first stop may move in a longitudinal direction between the open position and the closed position.

The holder may comprise a track to allow the first stop to move in a longitudinal direction. In other words, the first stop may be movable in a generally upstream direction and a generally downstream direction along a track. Movement of the first stop in an upstream direction may act to move the first stop into an open position and movement of the first stop in a downstream direction may act to move the first stop into a closed position. Alternatively, movement of the first stop in an upstream direction may act to move the first stop into a closed position and movement of the first stop in a downstream direction may act to move the first stop into an open position.

The first stop may pivot about a fixed point between the open position and the closed position.

This may advantageously provide a simple way for the first stop to move between the open position and the closed position. The first stop may be fixed to the housing of the holder.

Where the first stop is configured to pivot about a fixed point, the first stop may include a bent portion shaped to prevent the first follower member from moving the first stop when the user does not wish to move the first stop from the closed position to the open position.

The first stop may be fixed at one end to a portion of the holder. For example, the first stop may be fixed at one end to the housing. The first stop may comprise a portion of resiliently flexible material biased towards the closed position. Where this is the case, the movement of the first stop from the closed position to the open position may act against the bias of the portion of resiliently flexible material.

Where the holder comprises a sleeve, the sleeve may be further movable to an extraction position, the extraction position being further downstream than the downstream position.

In use, when an inhale article is depleted, the user may remove the inhaler article from the sleeve by pulling the inhaler article in a downstream direction. This action may move the sleeve from the downstream position to the extraction position, the extraction position being further downstream than the downstream position.

The first stop may be engageable with the sleeve such that the first stop is moved from the closed position to the open position when the sleeve is moved to the extraction position.

In use, when an inhaler article is removed from the sleeve, the sleeve may move to the extraction position. This action may advantageously move the first stop from the closed position to the open position. Once the first stop is in the open position, the first follower member may be able to move from the third position to the first position. In this way, removal of a depleted inhaler article from the holder may reset the holder allowing for activation of a new inhaler article. This may advantageously provide a simple way to reset the holder without the need for a user to actuate a separate reset mechanism.

The first stop may be fixed to the sleeve such that the first stop is unable to move relative to the sleeve. This would ensure that the movement of the sleeve causes a corresponding movement of the first stop.

The first stop may comprise a first stop protrusion. The sleeve may comprise a protrusion of the sleeve. As the sleeve is moved from the downstream position to the extraction position, the first stop protrusion may engage with the protrusion of the sleeve to urge the first stop from the closed position to the open position.

The sleeve may further comprise at least one projection located on the inner surface of the sleeve cavity for retaining an inhaler article.

The at least one projection may retain the inhaler article when the inhaler article is inserted into the sleeve. The inhaler article may be slightly compressible. Where this is the case, the provision of at least one projection may compress at least a portion of the inhaler article to grip and retain the inhaler article by forming an interference fit. This may advantageously prevent the inhaler article from falling out of the holder in use. The interference fit formed between the sleeve and the article may also advantageously act to move the sleeve from the downstream position to the extraction position when the inhaler article is removed from the holder. As set out above, this action may advantageously provide a simple means to reset the device to allow activation of a further inhaler article.

The at least one projection may comprise any number of projections. For example, the at least one projection may comprise 1, 2, 3, 4, 5, 8, 10, 12, or 14 projections. The at least one projection may comprise more than 10 projections.

The at least one projection may be elongate in shape. The at least one projection may be aligned with the longitudinal direction of the holder. Where this is the case, the at least one projection may extend along the full length of the sleeve.

The at least one projection may run in a direction perpendicular to the longitudinal direction of the holder. Where this is the case, the at least one projection may comprise a ring circumscribing the inner surface of the sleeve.

The holder may further comprise a downstream biasing element configured to urge the engagement member towards the downstream position.

When a user wishes to activate an inhaler article received in the holder, the user may push the inhaler article against the engagement member, or where present into the sleeve cavity, moving the engagement member from the downstream position to the upstream position to activate the inhaler article. Once a user releases the force on the inhaler article, the downstream biasing element may urge the engagement member towards the downstream position. In doing so, the first follower member may also be urged from the second position to the third position to prevent double activation of the inhaler article.

The downstream biasing element may be any biasing element. The downstream biasing element may comprise a compression spring acting between a portion of the holder housing and the engagement member, the movement of the engagement member in an upstream direction acting to compress the compression spring. The downstream biasing element may be connected to at least one of the housing and the engagement member. Alternatively, the downstream biasing element may simply be disposed between a position of the housing and the engagement member.

The holder may further comprise an upstream biasing element configured to urge the sleeve from the extraction position to the downstream position.

This may advantageously prevent the sleeve from moving freely between the extraction position and the downstream position. As set out above, the movement of the sleeve from the downstream position to the extraction position may cause the first stop to move from a closed position to an open position. It would therefore be undesirable for the sleeve to be in the extraction position during use of the inhaler article since the first stop could be open, allowing the first follower member to move from the third position to the first position and thereby not preventing the double activation of the inhaler article.

The upstream biasing element may be any biasing element. The upstream biasing element may comprise a compression spring. The upstream biasing element may comprise a compression spring acting between a portion of the holder housing and the sleeve, the movement of the sleeve in a downstream direction acting to compress the compression spring. The upstream biasing element may be connected to at least one of the housing and the sleeve. Alternatively, the upstream biasing element may simply be disposed between a position of the housing and the sleeve.

The holder may further comprise a first stop biasing element configured to urge the first stop to the closed position.

The provision of a first stop biasing element may advantageously ensure that the first stop remains in the closed position unless a user intends to reset the holder. The provision of a first stop biasing element may also ensure the orientation of the holder does not move the first stop between the open and closed positions. The first stop biasing element may be configured such that the movement of the first stop from the closed position to the open position acts against the first stop biasing element.

The first stop biasing element may act between a portion of the first stop and a portion of the holder housing. The housing may comprise a first protrusion configured to engage with the first stop biasing element. The first stop biasing element may be connected to at least one of the first stop and the holder housing. Alternatively, the first stop biasing element may simply be disposed between a portion of the first stop and a portion of the holder housing.

The first stop biasing element may be resiliently flexible. For example, the first stop biasing element may comprise a spring. The spring may be a compression spring. The spring may be a leaf spring. The first stop may be integrally formed with the first stop biasing element. For example, the first stop and the first stop biasing element may be formed from a single piece of polymeric material. The first stop biasing element may be fixed at one end to the holder housing.

One or more of the first stop biasing element and a resiliently flexible portion of the latch may be formed from a resiliently flexible material. Suitable resiliently flexible materials may include thermoplastic material. Suitable resiliently flexible materials may include polyetheretherketone, polyamide, acetal (polyactal or polyoxymethlene), polybutylene terephthalate, styrene acrylonitrile, or other materials that can be biased and elastically deformed. Suitable resiliently flexible materials may include acetal (polyactal or polyoxymethlene), and polyetheretherketone.

The engagement member may further comprise a second follower member and the housing may further comprise a second guide and a second latch, the second follower member being arranged to follow the second guide between a first position, a second position, and a third position, the second latch being arranged such that the second follower member may pass from the first position to the second position, but may not return to the first position without first passing the third position.

The provision of a second follower member, a second guide, and second latch may advantageously help to more reliably prevent the double activation of an inhaler article in substantially the same way as the first follower member, the first guide, and first latch as set out above.

The second follower member, second guide, and second latch as set out above may together form a second double activation prevention mechanism. The second double activation prevention mechanism may further comprise additional features corresponding to features of the first double activation prevention mechanism described above. For example, the holder may further comprise one or more of a second stop and a second stop biasing element.

The first stop and the second stop may be integrally formed. The first stop and the second stop may be formed from a single portion of resiliently flexible material. Where this is the case, the first stop and the second stop may together form a spring plate. The spring plate may be symmetrical such that both the first and second stops may move between the open and closed positons together. The spring plate may comprise a single portion of resiliently flexible material fixed to the housing at a point equidistant between the first guide and the second guide, and comprise a first and second arm which extend from the fixed position to the third position of the first guide and the third position of the second guide, respectively. In use, the first and second arms may deform to move the first and second stops from the closed positon to the open positon.

The second follower member, second guide, and second latch may be substantially identical to the first follower member, first guide, and first latch described above.

The second guide may be disposed substantially opposite to the first guide on the housing, and the second follower member may be disposed substantially opposite to the first follower member on the engagement member.

Locating the features of the second double activation prevention mechanism substantially opposite the first double activation prevention mechanism may advantageously provide balanced stopping force if a user tries to activate an inhaler article for a second time.

The engagement member may therefore comprise a body, the body being sized to be received in the housing cavity, the body having two protrusions on opposite sides of the body. The two protrusions forming the first and second follower members.

The holder may further comprise a piercing element extending into the housing from the upstream end of the housing. When the engagement member is in the downstream position, the engagement member may be positioned further downstream relative to the downstream end of the piercing element. When the engagement member is in the upstream position, the engagement member may be positioned further upstream relative to the downstream end of the piercing element in the upstream position.

Where the inhaler article comprises a powder capsule, the piercing element may activate the inhaler article by piercing the powder capsule. In use, when a user inserts a new inhaler article into the housing cavity and pushes it in an upstream direction, the engagement member is urged from the downstream position to the upstream position. In doing so, the downstream end of the piercing element extends further downstream than the engagement member allowing the piercing element to enter the inhaler article and pierce the powder capsule where present thereby activating the inhaler article.

The piercing element may be fixed to the upstream end of the housing and extend from the upstream end of the housing in a downstream direction.

The piercing element length may be any suitable length relative to the housing length. For example, the piercing element length may be about 25% to about 60%, or about 30% to about 50%, of the housing length.

The piercing element may be formed of a rigid material. The rigid material may be sufficiently rigid to pierce, puncture or activate a capsule contained within the inhaler article. The piercing element may be formed of a metal. The piercing element may be formed of stainless steel, such as 316 stainless steel, for example. The piercing element may be formed of a polymeric material. The piercing element may be formed of a fibre-reinforced polymeric material.

The engagement member may comprise an opening through which the piercing element may pass when the engagement member is moved from the downstream position to the upstream position.

The provision of an opening in the engagement member may advantageously allow the piercing element to pass through the engagement member to reach an inhaler article to activate the inhaler article when the engagement member is moved into the upstream position.

The opening may be aligned with the piercing element. The opening may be any size. The opening may have a diameter which is at least 50 percent larger than the diameter of the piercing element. This may advantageously allow the piercing element to pass through the opening even where the piercing element and opening are slightly misaligned.

The engagement member may be generally toroidal in shape.

The sleeve may comprise an opening through which the piercing element may pass when the sleeve is moved from the downstream position to the upstream position.

The provision of an opening in the sleeve may advantageously allow the piercing element to pass through the sleeve to reach an inhaler article to activate the inhaler article when the engagement member is moved into the upstream position.

The opening may be aligned with the piercing element and with the opening of the engagement member. The opening may be any size. The opening may have a diameter which is at least 50 percent larger than the diameter of the piercing element. This may advantageously allow the piercing element to pass through the opening even where the piercing element and opening are slightly misaligned.

According to the present invention, there is further provided an inhaler system comprising a holder according to the present invention. Furthermore, described is an inhaler article.

The inhaler article may be a dry powder inhaler article.

The inhaler article may resemble a smoking article or cigarette in size and shape. The inhaler article may have an elongated body extending along a longitudinal axis. The inhaler article may have a substantially uniform outer diameter. The inhaler article may have a circular cross-section that may be uniform along its length. The inhaler article may have an outer diameter in a range from 6 millimetres to 10 millimetres, or from 7 millimetres to 10 millimetres, or 7 millimetres to 9 millimetres, or 7 millimetres to 8 millimetres or about 7.2 millimetres. The inhaler article may have a length in a range from 40 millimetres to about 80 millimetres, or from 40 millimetres to 70 millimetres, or 40 millimetres to 50 millimetres, or 45 millimetres.

The inhaler article may have an open distal end or upstream-most end defined by a tubular element defining a central passage. The open central passage may define a cylindrical open aperture extending from a capsule cavity to the open distal end or upstream-most end of the inhaler article. The open tubular element defining an open central passage may have a length in a range from 3 millimetres to 12 millimetres, or from 3 millimetres to 7 millimetres, or from 4 millimetres to 6 millimetres, or 5 millimetres.

The open tubular element defining an open central passage may be formed of a cellulose material. The open tubular element defining an open central passage may be formed of a cellulose acetate material. Preferably the open tubular element is formed of a biodegradable material.

The inhaler article may comprise a filter element downstream of the capsule cavity. The filter element located downstream of the capsule cavity may extend from the capsule cavity to the mouthpiece end of the inhaler article.

The capsule cavity may define a cylindrical space configured to contain a capsule (the capsule may have an obround shape or a circular cross-section, for example). The capsule cavity may have a substantially uniform or uniform diameter along the length of the capsule cavity. The capsule cavity may have a fixed capsule cavity length. The capsule cavity has a cavity inner diameter, orthogonal to the longitudinal axis, and the capsule has a capsule outer diameter. The capsule cavity may be sized to contain an obround capsule. The capsule cavity may have a substantially circular or circular cross-section along the length of the capsule cavity. The capsule cavity may have a uniform inner diameter. The capsule may have an outer diameter that is about 80% to about 95% of the inner diameter of the capsule cavity. The configuration of the capsule cavity relative to the capsule may promote limited movement of the capsule during activation or piercing of the capsule.

The capsule cavity may be defined by an open tubular element. The open tubular element defining the capsule cavity may be joined between and in abutting alignment with the open tubular element defining the distal end of the inhaler article and the filter element. These elements may be joined with a wrapper. The open tubular element defining the capsule cavity may be formed of a biodegradable material, such as cardboard or paperboard.

The configuration of the capsule cavity relative to the capsule may promote the capsule to rotate with stability within the capsule cavity. The longitudinal axis of the capsule may rotate with stability co-axially with the longitudinal axis of the inhaler body during inhalation. The configuration of the capsule cavity relative to the capsule may promote the capsule to rotate with some shaking within the capsule cavity.

The capsule may be sealed within the inhaler article prior to consumption. The inhaler article may be contained within a sealed or airtight container or bag. The inhaler article may include one or more peelable or removable seal layers to cover the one or more air inlet channels or an air outlet or mouthpiece of the inhaler article.

The capsule may rotate about its longitudinal or central axis when air flows through the inhaler article. The capsule may have a shell formed of an airtight material that may be pierced or punctured by a piercing element that may be separate or combined with the inhaler. The capsule may be formed of a metallic or polymeric material that serves to keep contaminates out of the capsule but may be pierced or punctured by a piercing element prior to consumption of the nicotine particles within the capsule. The capsule may be formed of a polymer material. The polymer material may be hydroxypropylmethylcellulose (HPMC).

When activating the capsule, the holder of the present invention may form a single aperture through the capsule received in the capsule cavity.

The capsule may contain nicotine particles comprising nicotine (also referred to as "nicotine powder" or "nicotine particles") and optionally particles comprising flavour (also referred to as "flavour particles"). The capsule may contain a predetermined amount of nicotine particles and optional flavour particles. The capsule may contain enough nicotine particles to provide at least 2 inhalations or "puffs", or at least about 5 inhalations or "puffs", or at least about 10 inhalations or "puffs". The capsule may contain enough nicotine particles to provide from about 5 to about 50 inhalations or "puffs", or from about 10 to about 30 inhalations or "puffs". Each inhalation or "puff" may deliver from about 0.1 mg to about 3 mg of nicotine particles to the lungs of the user or from about 0.2 mg to about 2 mg of nicotine particles to the lungs of the user or about 1 mg of nicotine particles to the lungs of the user.

The nicotine particles may have any useful concentration of nicotine based on the particular formulation employed. The nicotine particles may have at least about 1%wt nicotine up to about 30%wt nicotine, or from about 2%wt to about 25%wt nicotine, or from about 3%wt to about 20%wt nicotine, or from about 4%wt to about 15%wt nicotine, or from about 5%wt to about 13%wt nicotine. Preferably, about 50 to about 150 micrograms of nicotine may be delivered to the lungs of the user with each inhalation or "puff".

When flavour particles are blended or combined with the nicotine particles within the capsule, the flavour particles may be present in an amount that provides the desired flavour to each inhalation or "puff" delivered to the user.

The nicotine particles may have any useful size distribution for inhalation delivery preferentially into the lungs of a user. The capsule may include particles other than the nicotine particles. The nicotine particles and the other particles may form a powder system.

The capsule may hold or contain at least about 5 mg of a dry powder (also referred to as a powder system) or at least about 10 mg of a dry powder. The capsule may hold or contain less than about 900 mg of a dry powder, or less than about 300 mg of a dry powder, or less than about 150 mg of a dry powder. The capsule may hold or contain from about 5 mg to about 300 mg of a dry powder, or from about 10 mg to about 200 mg of a dry powder, or from about 25 mg to about 100 mg of a dry powder.

The dry powder or powder system may have at least about 40%, or at least about 60%, or at least about 80%, by weight of the powder system comprised in nicotine particles having a particle size of about 5 micrometers or less, or in a range from about 1 micrometer to about 5 micrometres.

The particles comprising nicotine may have a mass median aerodynamic diameter of about 5 micrometres or less, or in a range from about 0.5 micrometres to about 4 micrometres, or in a range from about 1 micrometres to about 3 micrometres or in a range from about 1.5 micrometres to about 2.5 micrometres. The mass median aerodynamic diameter is preferably measured with a cascade impactor.

The particles comprising flavour may have a mass median aerodynamic diameter of about 20 micrometres or greater, or about 50 micrometres or greater, or in a range from about 50 to about 200 micrometres, or from about 50 to about 150 micrometres. The mass median aerodynamic diameter is preferably measured with a cascade impactor.

The dry powder may have a mean diameter of about 60 micrometres or less, or in a range from about 1 micrometres to about 40 micrometres, or in a range from about 1.5 micrometres to about 25 micrometres. The mean diameter refers to the mean diameter per mass and is preferably measured by laser diffraction, laser diffusion or an electronic microscope.

Nicotine in the powder system or nicotine particles may be a pharmaceutically acceptable free-base nicotine, or nicotine salt or nicotine salt hydrate. Useful nicotine salts or nicotine salt hydrates include nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. The compound combining with nicotine to form the salt or salt hydrate may be chosen based on its expected pharmacological effect.

The nicotine particles preferably include an amino acid. Preferably the amino acid may be leucine such as L-leucine. Providing an amino acid such as L-leucine with the particles comprising nicotine, may reduce adhesion forces of the particles comprising nicotine and may reduce attraction between nicotine particles and thus reduce agglomeration of nicotine particles. Similarly, adhesion forces to particles comprising flavour may also be reduced thus agglomeration of nicotine particles with flavour particles is also reduced. The powder system described herein thus may be a free-flowing material and possess a stable relative particle size of each powder component even when the nicotine particles and the flavour particles are combined.

Preferably, the nicotine may be a surface modified nicotine salt where the nicotine salt particle comprises a coated or composite particle. A preferred coating or composite material may be L-leucine. One particularly useful nicotine particle may be nicotine bitartrate with L-leucine.

The powder system may include a population of flavour particles. The flavour particles may have any useful size distribution for inhalation delivery selectively into the mouth or buccal cavity of a user.

The powder system may have at least about 40%, or at least about 60%, or at least about 80%, by weight of the population of flavour particles of the powder system comprised in particles having a particle size of about 20 micrometres or greater. The powder system may have at least about 40% or at least about 60%, or at least about 80%, by weight of the population of flavour particles of the powder system comprised in particles having a particle size of about 50 micrometres or greater. The powder system may have at least about 40% or at least about 60%, or at least about 80%, by weight of the population of flavour particles of the powder system comprised in particles having a particle size in a range from about 50 micrometer to about 150 micrometres.

The particles comprising flavour may include a compound to reduce adhesion forces or surface energy and resulting agglomeration. The flavour particle may be surface modified with an adhesion reducing compound to form a coated flavour particle. One preferred adhesion reducing compound may be magnesium stearate. Providing an adhesion reducing compound such as magnesium stearate with the flavour particle, especially coating the flavour particle, may reduce adhesion forces of the particles comprising flavour and may reduce attraction between flavour particles and thus reduce agglomeration of flavour particles. Thus, agglomeration of flavour particles with nicotine particles may also be reduced. The powder system described herein thus may possess a stable relative particle size of the particles comprising nicotine and the particles comprising flavour even when the nicotine particles and the flavour particles are combined. The powder system preferably may be free flowing.

Conventional formulations for dry powder inhalation contain carrier particles that serve to increase the fluidization of the active particles since the active particles may be too small to be influenced by simple airflow though the inhaler. The powder system may comprise carrier particles. These carrier particles may be a saccharide such as lactose or mannitol that may have a particle size greater than about 50 micrometres. The carrier particles may be utilized to improve dose uniformity by acting as a diluent or bulking agent in a formulation.

The powder system utilized with the nicotine powder delivery system described herein may be carrier-free or substantially free of a saccharide such as lactose or mannitol. Being carrier-free or substantially free of a saccharide such as lactose or mannitol may allow the nicotine and to be inhaled and delivered to the user's lungs at inhalation or airflow rates that are similar to typical smoking regime inhalation or airflow rates.

The nicotine particles and a flavour may be combined in a single capsule. As described above, the nicotine particles and a flavour may each have reduced adhesion forces that result in a stable particle formulation where the particle size of each component does not substantially change when combined. Alternatively, the powder system includes nicotine particles contained within a single capsule and the flavour particles contained within a second capsule.

The nicotine particles and flavour particles may be combined in any useful relative amount so that the flavour particles are detected by the user when consumed with the nicotine particles. Preferably the nicotine particles and a flavour particles form at least about 90%wt or at least about 95%wt or at least about 99%wt or 100%wt of the total weight of the powder system.

The inhaler and inhaler system may be less complex and have a simplified airflow path as compared to conventional dry powder inhalers. Advantageously, rotation of the capsule within the inhaler body aerosolizes the nicotine particles or powder system and may assist in maintaining a free-flowing powder. Thus, the inhaler article may not require the elevated inhalation rates typically utilized by conventional inhalers to deliver the nicotine particles described above deep into the lungs.

The inhaler article may use a flow rate of less than about 5 L/min or less than about 3 L/min or less than about 2 L/min or about 1.6 L/min. Preferably, the flow rate may be in a range from about 1 L/min to about 3 L/min or from about 1.5 L/min to about 2.5 L/min. Preferably, the inhalation rate or flow rate may be similar to that of Health Canada smoking regime, that is, about 1.6 L/min.

The inhaler system may be used by a consumer like smoking a conventional cigarette or vaping an electronic cigarette. Such smoking or vaping may be characterized by two steps: a first step during which a small volume containing the full amount of nicotine desired by the consumer is drawn into the mouth cavity, followed by a second step during which this small volume comprising the aerosol comprising the desired amount of nicotine is further diluted by fresh air and drawn deeper into the lungs. Both steps are controlled by the consumer. During the first inhalation step the consumer may determine the amount of nicotine to be inhaled. During the second step, the consumer may determine the volume for diluting the first volume to be drawn deeper into the lungs, maximizing the concentration of active agent delivered to the airway epithelial surface. This smoking mechanism is sometimes called "puff-inhale-exhale".

The invention is defined in the claims.

Examples will now be further described with reference to the figure in which:
Figure 1 shows a transparent profile view of an inhaler system according to the present invention comprising a holder and an inhaler article.
Figure 2 shows a section view in profile of an inhaler system according to the present invention comprising a holder and an inhaler article.
Figure 3 shows a schematic view of a portion of the holder according to the present invention showing the first double activation prevention mechanism.
Figure 4 shows a detailed view of a portion of the holder according to the present invention showing the first double activation prevention mechanism.
Figure 5 shows a detailed view of a portion of the holder according to the present invention showing the first stop.
Figure 6 shows a detailed section view of a portion of the holder according to the present invention showing the first stop.
Figure 7 shows a perspective view of the engagement member of the present invention.
Figure 8 shows a section plan view of a portion of the holder according to the present invention showing the engagement member.
Figure 9 shows a perspective view of the engagement member of the present invention.
Figure 10 shows a detailed section view of a portion of an inhaler system according to the present invention.

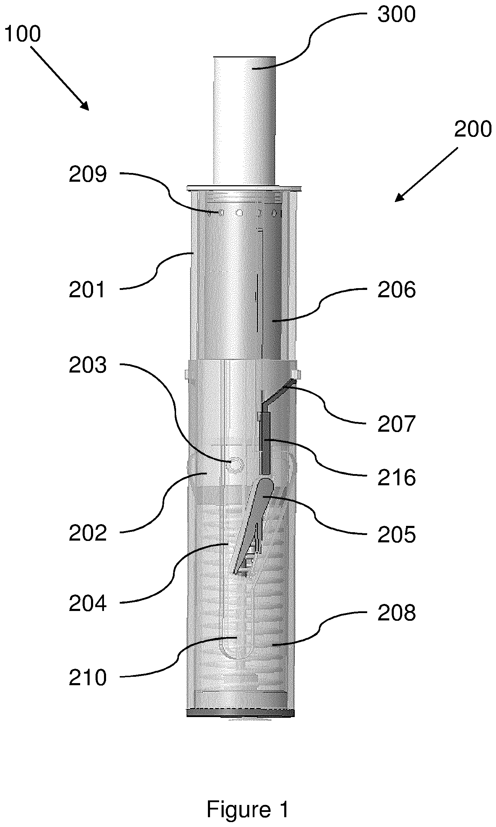

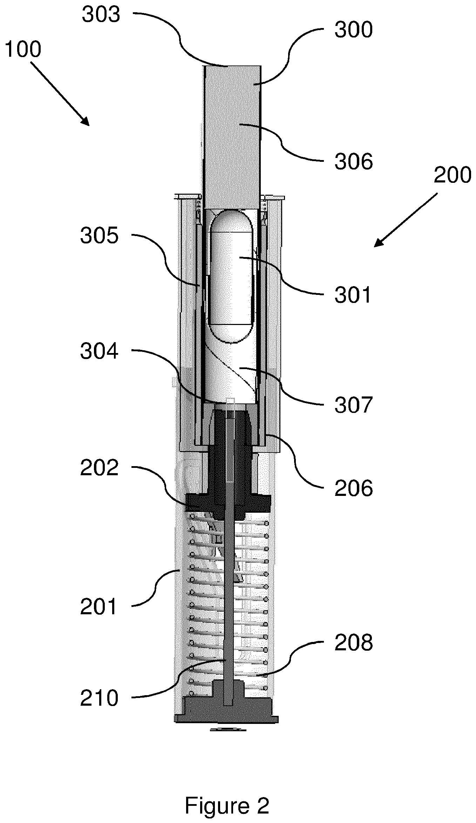

Referring to Figure 1 and Figure 2, the inhaler system 100 comprises a holder 200 and an inhaler article 300. The holder 200 comprises a housing 201 comprising a housing cavity. The holder 200 further comprises an engagement member 202 located within the housing cavity. The engagement member 202 is movable relative to the housing along the longitudinal axis of the holder 200 between an upstream position and a downstream position. The engagement member 202 comprises a first follower member 203. The housing 201 comprises a first guide 204 and a first latch 205. The first follower member 203 is arranged to follow the first guide 204 between a first position 221, a second position 222, and a third position 223. The first latch 205 is arranged such that the first follower member 203 may pass from the first position 221 to the second position 222, but may not return to the first position 221 without first passing the third position 223.

The housing 201 comprises a hollow cylindrical tube closed at an upstream end and open at a downstream end.

The engagement member 202 is rotatable about the longitudinal axis of the holder 200.

The holder 200 further comprises a sleeve 206. The sleeve 206 is disposed at least partially within the housing cavity and is positioned downstream of the engagement member 202. The sleeve 206 is generally cylindrical in shape with an open downstream end and a closed upstream end. The sleeve 206 comprises a sleeve cavity for receiving an inhaler article. The external diameter of the sleeve 206 is substantially the same as the diameter of the engagement member 202. The sleeve 206 and the engagement member 202 are arranged in the housing cavity such that the upstream end of the sleeve 206 is able to abut the downstream end of the engagement member 202. Where the sleeve 206 and the engagement member 202 are in contact, movement of the engagement member 202 from the upstream position to the downstream position causes the sleeve 206 to move from a corresponding upstream position to a corresponding downstream position. Similarly, the movement of the sleeve 206 from a downstream position to an upstream position causes the engagement member 202 to move from a downstream position to an upstream.

The sleeve 206 is further movable to an extraction position. The sleeve 206 is further downstream in the extraction position than it is in the downstream position.

The inner diameter of the sleeve cavity is slightly larger than the external diameter of the inhaler article 300 intended to be received in the sleeve cavity. The inner surface of the sleeve comprises a plurality of projections extending into the sleeve cavity. The plurality of projections comprises twelve elongate projections extending along a portion of the length of the inner surface of the sleeve in a longitudinal direction.

The sleeve 206 further comprises a plurality of air inlets 209 circumscribing the sleeve 206 close to the downstream end of the sleeve 206. The plurality of air inlets 209 allow air to enter the sleeve cavity at a location upstream of the downstream end of the sleeve 206.

The first follower member 203 is integrally formed with the remainder of the engagement member 202. Movement of the engagement member 202 between the downstream and upstream positions causes a corresponding movement of the first follower member 203. More specifically, when the engagement member 202 is in the downstream position, this corresponds to the first follower member 203 being in either the first position 221 or the third position 223. When the engagement member 202 is in the upstream position, this corresponds to the first follower member 203 being in the second position 222.

The first guide 204 comprises a groove on the inner surface of the housing 201. The groove of the first guide 204 has a diameter generally corresponding to the diameter of the first follower member 203 such that the groove can act as a guide. The first guide 204 comprises a ledge at the third position 223. The ledge is positioned to prevent the first follower member from passing from the third position 223 back to the second position 222 by moving in an upstream direction.

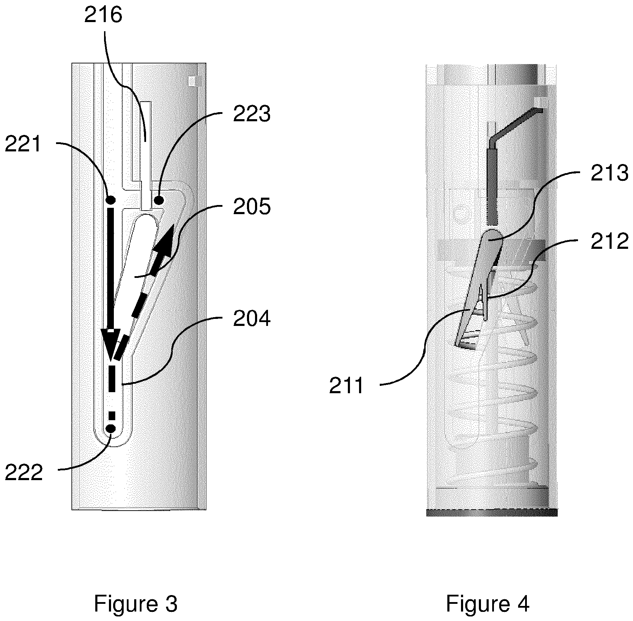

Referring to Figure 3, the first position 221 of the first follower member in the guide 204 is located further downstream than the second position 222. The first position 221 and the second position 222 are substantially aligned along the longitudinal direction of the holder. The third position 223 is level with the first position 221 in the longitudinal direction, but is offset from both the first 221 and second positions 223. The movement of the first follower member 203 from the first position 221 to the second position 222, and then to the third position 223 is indicated by arrows in Figure 3.

Referring to Figure 4, the first latch 205 comprises an inflexible portion 211 and a resiliently flexible portion 212. The first latch 205 is connected to the housing 201 at a pivot point 213 about which the first latch 205 is able to rotate. The inflexible portion 211 and a resiliently flexible portion 212 are formed from a single piece of polymeric material. The resiliently flexible portion 212 acts between the inflexible portion 211 and a portion of the housing to urge the inflexible portion 211 into a position which extends across the first guide 204 between the first position 221 and the second position 222. When the first follower member 203 moves from the first position 221 to the upstream position 222, it pushes past the inflexible portion 211 against the action of the resiliently flexible portion 212.

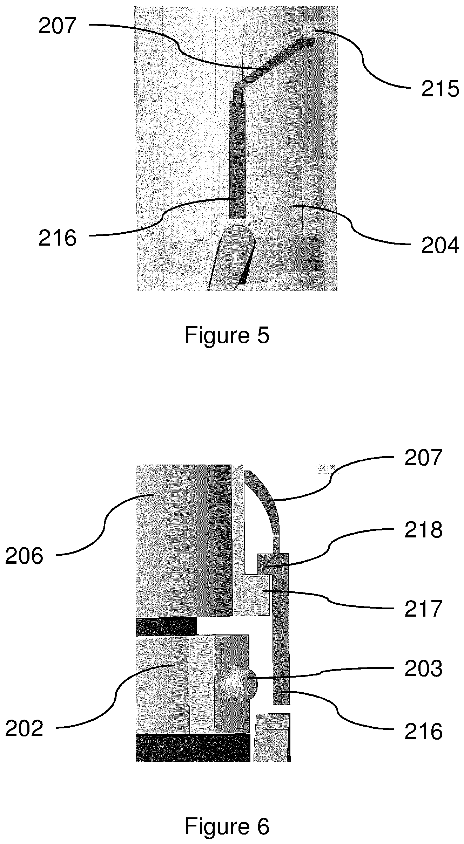

Referring to Figures 5 and 6, the holder further comprises a first stop 216. The first stop 216 is located generally between the third position 223 and the first position 221 and is movable between an open and a closed position. In the open position, the first follower member 203 is able to pass from the third position 223 to the first position 221. In the closed position, the first stop 216 prevents the first follower member 203 from passing from the third position 223 to the first position 221. The first stop 216 moves in the longitudinal direction between the open and closed positions. The first stop 216 comprises a first stop biasing element 207 to urge the first stop into the closed position. The first stop biasing element 207 is integrally formed with the first stop 216 from a single piece of polymeric material. The first stop biasing element 207 acts between the first stop 216 and a first protrusion 215 on the housing 201. The first stop biasing element 207 is resiliently flexible.

The first stop 216 further comprises a first stop protrusion 218 which is engagable with a protrusion 217 of the sleeve 206. When the sleeve 206 is moved in a downstream direction, the first stop protrusion 218 engages with the protrusion 217 of the sleeve 206 to move the first stop 216 from the closed position to the open position against the action of the first stop biasing element.

The first follower member 203, the first guide 204, the first latch 205, and the first stop 216 together form a first double activation prevention mechanism.

The holder 200 further comprises a second double activation mechanism disposed on the opposite side of the holder 200. The second double activation mechanism comprises features identical to the features of the first double activation mechanism.

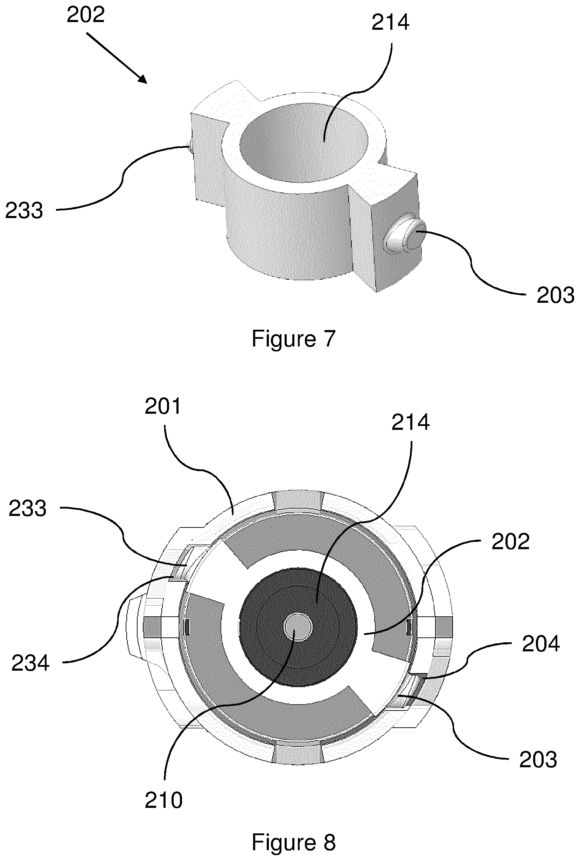

Referring to Figures 7 and 8, the engagement member 202 comprises a central opening 214 and includes the first follower member 203 and a second follower member 233 disposed on opposite sides of the engagement member 202. The sleeve 206 further comprises an opening (not shown) which is aligned with the opening 214 of the engagement member 202.

Figure 8 shows the engagement member 202 in situ where the first follower member 203 is shown in the first guide 204, and the second follower member 233 is shown in a second guide 234 disposed on the opposite side of the holder 200.

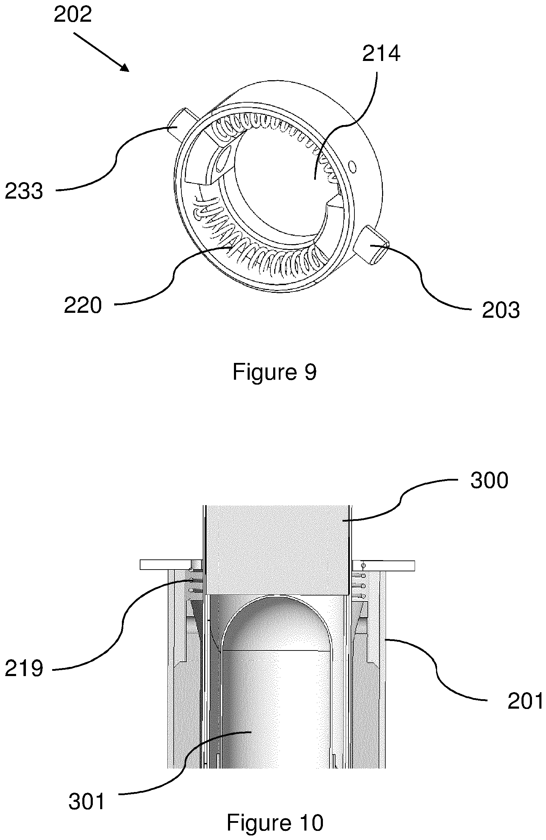

Referring to Figure 9, the engagement member 202 further comprises an engagement member biasing element 220. The engagement member biasing element 220 comprises a compression spring disposed at least partially within the engagement member 202. The engagement member biasing element 220 is configured to apply a torsional force to the engagement member 202 to urge the first follower member 203 from the third position 223 to the first position 221, and to urge the second follower member 233 from the third position to the first position such that when the first stop 216 and second stop are moved to the open positions, the engagement member 202 rotates to move the first and second follower members 203, 233 from the third positions to the first positions.

As best shown in Figure 2, the holder 200 further comprises a downstream biasing element 208 to urge the engagement member 202 towards the downstream position. The downstream biasing element 208 comprises a compression spring which acts between the upstream end of the housing 201 and the upstream end of the engagement member 202.

As best shown in Figure 10, the holder 200 further comprises an upstream biasing element 219 to urge the sleeve 206 from the extraction position towards the upstream position. The upstream biasing element 219 comprises a compression spring which acts between the downstream end of the housing 201 and the downstream end of the sleeve 206.

The holder 200 further comprises a piercing element 210. The piercing element 210 is attached to the upstream end of the housing 201 and extends into the housing cavity. The piercing element 210 is configured such that when the engagement member 202 is in the upstream position, the piercing element 210 extends through the opening 214 of the engagement member and the opening of the sleeve 206. When the engagement member 202 is in the downstream position, the engagement member 202 is downstream of the piercing element 210 such that the piercing element 210 does not extend though the opening 214.

The inhaler system 100 further comprises an inhaler article 300. As best shown in Figure 2, the inhaler article 300 extends from a mouthpiece end 303 to an upstream end 304. A capsule cavity 305 is disposed within the inhaler article 300 and is bounded downstream by a filter element 306 and bounded upstream by a tubular element 307 defining a central passage. The central passage forms an air-inlet aperture. A capsule 301 is disposed within the capsule cavity 305.

In use, an inhaler article 300 is inserted into the sleeve 206 and is pushed such that the sleeve 206 and the engagement member 202 move from the downstream position to the upstream position. This causes the piercing element 210 to pass through the opening 214 in the engagement member 202 and through the opening in the sleeve to pierce the capsule 301 of the inhaler article 300 thereby activating the inhaler article. The movement of the engagement member 202 from the downstream position to the upstream position also causes the first (and second) follower member 203 to move from the first position 221 to the second position 222. As the first follower member 203 moves from the first position 221 to the second position 222, it pushes the inflexible portion 211 of the latch 205 aside against the action of the resiliently flexible portion 212 of the latch 205.

Once the inhaler article 300 is activated, it is released. The downstream biasing element 208 then moves the engagement member 202 and the sleeve 206 from the upstream position to the downstream position. As this happens, the first follower member 203 moves from the second position 222 to the third position 223. The first latch prevents the first follower member 203 from passing directly back to the first position 221. In the third position 223, the first follower member 203 is unable to return to the second position 222 because of the ledge of the first guide 204, and is unable to advance to the to the first position 221 because of the first stop 216.

In use, air enters the sleeve 206 through the plurality of air inlets 209. This air enters the inhaler article 300, becomes entrained with the powder from the capsule 301 before leaving the inhaler article 300 through the mouthpiece end 303 of the inhaler article 300.

Once the inhaler article 300 is depleted, it is removed from the holder 200 by pulling it in a downstream direction. The plurality of projections on the inner surface of the sleeve cavity act to grip the inhaler article 300 by forming an interference fit. As a result, the sleeve 206 is pulled from the downstream position to the extraction position against the action of the upstream biasing element 219. As the sleeve 206 is moved to the extraction position, the protrusion 217 of the sleeve engages with the first stop protrusion 218 to move the first stop 216 from the closed position to the open position against the action of the first stop biasing element 207. Once the first stop 216 opens, the action of the engagement member biasing element 220 acts to rotate the engagement member 202 such that the first follower member 203 moves from the third position 223 back to the first position 221. This resets the double activation prevention mechanism.

Once the inhaler article 300 has been removed from the holder 200, the upstream biasing element 219 urges the sleeve 206 from the extraction position to the downstream position and the first stop biasing element 207 urges the first stop 216 from the open position to the closed position.

## Claims

1. A holder (200) for an inhaler article (300), the holder (200) comprising:
a housing (201) comprising a housing cavity, and
an engagement member (202) located at least partially within the housing cavity and being movable relative to the housing (201) between an upstream position and a downstream position, the engagement member (202) comprising a first follower member (203),
the housing (201) comprising a first guide (204) and a first latch (205), the first follower member (203) being arranged to follow the first guide (204) between a first position (221), a second position (222), and a third position (223), the first latch (205) being arranged such that the first follower member (203) may pass from the first position (221) to the second position (222), but may not return to the first position (221) without first passing the third position (223), the first latch (205) preventing movement of the first follower member (203) from the second position (222) directly back to the first position (221).

2. A holder (200) for an inhaler article (300) according to claim 1, further comprising a sleeve (206) comprising a sleeve cavity for receiving an inhaler article (300), the sleeve (206) being located at least partially within the housing cavity movable relative to the housing (201) between an upstream position and a downstream position, the upstream and downstream positions corresponding to the upstream and downstream positions of the engagement member (202).

3. A holder (200) for an inhaler article (300) according to claim 2, wherein the engagement member (202) and the sleeve (206) are sized and positioned in the housing cavity such that the movement of the sleeve (206) in an upstream direction acts to urge the engagement member (202) in an upstream direction, and the movement of the engagement member (202) in a downstream direction acts to urge the engagement member (202) in a downstream direction.

4. A holder (200) for an inhaler article (300) according to any preceding claim, wherein the engagement member (202) is rotatable about an axis of the holder (200) relative to the housing (201).

5. A holder (200) for an inhaler article (300) according to claim 2 or claim 3, wherein the engagement member (202) is rotatable about an axis of the holder (200) relative to the sleeve (206).

6. A holder (200) for an inhaler article (300) according to any preceding claim, wherein the first guide (204) is configured such that, once the first follower member (203) is in the third position (223), it is unable to return to the second position (222).

7. A holder (200) for an inhaler article (300) according to any preceding claim, wherein the movement of the engagement member (202) from the downstream position to the upstream position causes the first follower member (203) to move from the first position (221) to the second position (222) along the first guide (204).

8. A holder (200) for an inhaler article (300) according to any preceding claim, wherein the movement of the engagement member (202) from the upstream position to the downstream position causes the first follower member (203) to move from the second position (222) to the third position (223) along the first guide (204).

9. A holder (200) for an inhaler article (300) according to claim 2, wherein the housing (201) comprises a first stop (216) between the third position (223) and first position (221) arranged to prevent the first follower member (203) moving from the third position (223) to the first position (221).

10. A holder (200) for an inhaler article (300) according to claim 9, wherein the first stop (216) is movable between a closed position in which the first stop (216) prevents the first follower member (203) from moving from the third position (223) to the first position (221), and an open position in which the first follower member (203) is able to move from the third position (223) to the first position (221).

11. A holder (200) for an inhaler article (300) according to any preceding claim, further comprising an engagement member biasing element (220) configured to urge the first follower member (203) from the third position (223) to the first position (221).

12. A holder (200) for an inhaler article (300) according to any preceding claim, wherein the engagement member biasing element (220) is a spring configured to apply a torsional force to the engagement member (202).

13. A holder (200) for an inhaler article (300) according to claim 9 or claim 10, wherein the sleeve (206) is further movable to an extraction position, the extraction position being further downstream than the downstream position.

14. A holder (200) for an inhaler article (300) according to claim 13, wherein the first stop (216) is engageable with the sleeve (206) such that the first stop (216) is moved from the closed position to the open position when the sleeve (206) is moved to the extraction position.

15. An inhaler system (100) comprising a holder (200) according to any preceding claim and an inhaler article (300).

## Patentansprüche

1. Halterung (200) für einen Inhalatorartikel (300), die Halterung (200) umfassend:
ein Gehäuse (201), umfassend einen Gehäusehohlraum, und
ein Eingriffselement (202), das wenigstens teilweise innerhalb des Gehäusehohlraums angeordnet ist und in Bezug auf das Gehäuse (201) zwischen einer vorgelagerten Position und einer nachgelagerten Position beweglich ist, wobei das Eingriffselement (202) ein erstes Mitnehmerelement (203) umfasst,
das Gehäuse (201) umfassend eine erste Führung (204) und eine erste Verriegelung (205), das erste Mitnehmerelement (203) angeordnet, um der ersten Führung (204) zwischen einer ersten Position (221), einer zweiten Position (222) und einer dritten Position (223) zu folgen, die erste Verriegelung (205) angeordnet, sodass das erste Mitnehmerelement (203) von der ersten Position (221) zu der zweiten Position (222) übergehen kann, jedoch nicht in die erste Position (221) zurückkehren kann, ohne zuvor die dritte Position (223) zu durchlaufen, die erste Verriegelung (205) eine Bewegung des ersten Mitnehmerelements (203) von der zweiten Position (222) direkt zurück in die erste Position (221) verhindernd.

2. Halterung (200) für einen Inhalatorartikel (300) nach Anspruch 1, ferner umfassend eine Hülse (206), umfassend einen Hülsenhohlraum für ein Aufnehmen eines Inhalatorartikels (300), die Hülse (206) ist wenigstens teilweise innerhalb des Gehäusehohlraums angeordnet, der in Bezug auf das Gehäuse (201) zwischen einer vorgelagerten Position und einer nachgelagerten Position beweglich ist, die vorgelagerte und die nachgelagerte Positionen entsprechen den vorgelagerten und nachgelagerten Positionen des Eingriffselements (202).

3. Halterung (200) für einen Inhalatorartikel (300) nach Anspruch 2, wobei das Eingriffselement (202) und die Hülse (206) derart bemessen und in dem Gehäusehohlraum positioniert sind, dass die Bewegung der Hülse (206) in einer vorgelagerten Richtung bewirkt, dass das Eingriffselement (202) in eine vorgelagerte Richtung gedrängt wird, und die Bewegung des Eingriffselements (202) in einer nachgelagerten Richtung bewirkt, dass das Eingriffselement (202) in eine nachgelagerte Richtung gedrängt wird.

4. Halterung (200) für einen Inhalatorartikel (300) nach einem beliebigen vorhergehenden Anspruch, wobei das Eingriffselement (202) um eine Achse der Halterung (200) in Bezug auf das Gehäuse (201) drehbar ist.

5. Halterung (200) für einen Inhalatorartikel (300) nach Anspruch 2 oder Anspruch 3, wobei das Eingriffselement (202) um eine Achse der Halterung (200) in Bezug auf die Hülse (206) drehbar ist.

6. Halterung (200) für einen Inhalatorartikel (300) nach einem beliebigen vorhergehenden Anspruch, wobei die erste Führung (204) derart eingerichtet ist, dass das erste Mitnehmerelement (203), sobald es sich in der dritten Position (223) befindet, nicht in die zweite Position (222) zurückkehren kann.

7. Halterung (200) für einen Inhalatorartikel (300) nach einem beliebigen vorhergehenden Anspruch, wobei die Bewegung des Eingriffselements (202) von der nachgelagerten Position in die vorgelagerte Position bewirkt, dass sich das erste Mitnehmerelement (203) von der ersten Position (221) in die zweite Position (222) entlang der ersten Führung (204) bewegt.

8. Halterung (200) für einen Inhalatorartikel (300) nach einem beliebigen vorhergehenden Anspruch, wobei die Bewegung des Eingriffselements (202) von der vorgelagerten Position in die nachgelagerte Position bewirkt, dass sich das erste Mitnehmerelement (203) von der zweiten Position (222) in die dritte Position (223) entlang der ersten Führung (204) bewegt.

9. Halterung (200) für einen Inhalatorartikel (300) nach Anspruch 2, wobei das Gehäuse (201) einen ersten Anschlag (216) zwischen der dritten Position (223) und der ersten Position (221) umfasst, der angeordnet ist, um zu verhindern, dass sich das erste Mitnehmerelement (203) von der dritten Position (223) in die erste Position (221) bewegt.

10. Halterung (200) für einen Inhalatorartikel (300) nach Anspruch 9, wobei der erste Anschlag (216) zwischen einer geschlossenen Position, in der der erste Anschlag (216) das erste Mitnehmerelement (203) daran hindert, sich von der dritten Position (223) in die erste Position (221) zu bewegen, und einer offenen Position, in der sich das erste Mitnehmerelement (203) von der dritten Position (223) in die erste Position (221) bewegen kann, bewegbar ist.

11. Halterung (200) für einen Inhalatorartikel (300) nach einem beliebigen vorhergehenden Anspruch, ferner umfassend ein Eingriffselement-Vorspannelement (220), das dazu eingerichtet ist, das erste Mitnehmerelement (203) aus der dritten Position (223) in die erste Position (221) zu drängen.

12. Halterung (200) für einen Inhalatorartikel (300) nach einem beliebigen vorhergehenden Anspruch, wobei das Eingriffselement-Vorspannelement (220) eine Feder ist, die dazu eingerichtet ist, eine Torsionskraft auf das Eingriffselement (202) auszuüben.

13. Halterung (200) für einen Inhalatorartikel (300) nach Anspruch 9 oder Anspruch 10, wobei die Hülse (206) ferner in eine Entnahmeposition bewegbar ist, wobei die Entnahmeposition weiter nachgelagert ist als die nachgelagerte Position.

14. Halterung (200) für einen Inhalatorartikel (300) nach Anspruch 13, wobei der erste Anschlag (216) mit der Hülse (206) in Eingriff bringbar ist, sodass der erste Anschlag (216) von der geschlossenen Position in die offene Position bewegt wird, wenn die Hülse (206) in die Entnahmeposition bewegt wird.

15. Inhalatorsystem (100), umfassend eine Halterung (200) nach einem beliebigen vorhergehenden Anspruch und einen Inhalatorartikel (300).

## Revendications

1. Support (200) pour un article inhalateur (300), le support (200) comprenant :
un logement (201) comprenant une cavité de logement, et
un organe de mise en prise (202) situé au moins partiellement au sein de la cavité de logement et étant mobile par rapport au logement (201) entre une position amont et une position aval, l'organe de mise en prise (202) comprenant un premier organe suiveur (203),
le logement (201) comprenant un premier guide (204) et un premier loquet (205), le premier organe suiveur (203) étant agencé pour suivre le premier guide (204) entre une première position (221), une deuxième position (222) et une troisième position (223), le premier loquet (205) étant agencé de telle sorte que le premier organe suiveur (203) puisse passer de la première position (221) à la deuxième position (222), mais ne peut pas revenir à la première position (221) sans passer au préalable la troisième position (223), le premier loquet (205) empêchant le déplacement du premier organe suiveur (203) de la deuxième position (222) directement de retour à la première position (221).

2. Support (200) pour un article inhalateur (300) selon la revendication 1, comprenant en outre un manchon (206) comprenant une cavité de manchon pour recevoir un article inhalateur (300), le manchon (206) étant situé au moins partiellement au sein de la cavité de logement mobile par rapport au logement (201) entre une position amont et une position aval, les positions amont et aval correspondant aux positions amont et aval de l'organe de mise en prise (202).

3. Support (200) pour un article inhalateur (300) selon la revendication 2, dans lequel l'organe de mise en prise (202) et le manchon (206) sont dimensionnés et positionnés dans la cavité de logement de telle sorte que le déplacement du manchon (206) dans une direction amont agit pour pousser l'organe de mise en prise (202) dans une direction amont, et le déplacement de l'organe de mise en prise (202) dans une direction aval agit pour pousser l'organe de mise en prise (202) dans une direction aval.

4. Support (200) pour un article inhalateur (300) selon l'une quelconque des revendications précédentes, dans lequel l'organe de mise en prise (202) peut tourner autour d'un axe du support (200) par rapport au logement (201).

5. Support (200) pour un article inhalateur (300) selon la revendication 2 ou la revendication 3, dans lequel l'organe de mise en prise (202) peut tourner autour d'un axe du support (200) par rapport au manchon (206).

6. Support (200) pour un article inhalateur (300) selon l'une quelconque des revendications précédentes, dans lequel le premier guide (204) est configuré de telle sorte que, une fois que le premier organe suiveur (203) est dans la troisième position (223), il est incapable de revenir à la deuxième position (222).

7. Support (200) pour un article inhalateur (300) selon l'une quelconque des revendications précédentes, dans lequel le déplacement de l'organe de mise en prise (202) de la position aval à la position amont amène le premier organe suiveur (203) à se déplacer de la première position (221) à la deuxième position (222) le long du premier guide (204).

8. Support (200) pour un article inhalateur (300) selon l'une quelconque des revendications précédentes, dans lequel le déplacement de l'organe de mise en prise (202) de la position amont à la position aval amène le premier organe suiveur (203) à se déplacer de la deuxième position (222) à la troisième position (223) le long du premier guide (204).

9. Support (200) pour un article inhalateur (300) selon la revendication 2, dans lequel le logement (201) comprend une première butée (216) entre la troisième position (223) et la première position (221) agencée pour empêcher le premier organe suiveur (203) de se déplacer de la troisième position (223) à la première position (221).

10. Support (200) pour un article inhalateur (300) selon la revendication 9, dans lequel la première butée (216) est mobile entre une position fermée dans laquelle la première butée (216) empêche le premier organe suiveur (203) de se déplacer de la troisième position (223) à la première position (221), et une position ouverte dans laquelle le premier organe suiveur (203) est capable de se déplacer de la troisième position (223) à la première position (221).

11. Support (200) pour un article inhalateur (300) selon l'une quelconque des revendications précédentes, comprenant en outre un élément de sollicitation d'organe de mise en prise (220) configuré pour pousser le premier organe suiveur (203) de la troisième position (223) à la première position (221).

12. Support (200) pour un article inhalateur (300) selon l'une quelconque des revendications précédentes, dans lequel l'élément de sollicitation d'organe de mise en prise (220) est un ressort configuré pour appliquer une force de torsion à l'organe de mise en prise (202).

13. Support (200) pour un article inhalateur (300) selon la revendication 9 ou la revendication 10, dans lequel le manchon (206) est en outre mobile vers une position d'extraction, la position d'extraction étant en outre plus en aval que la position aval.

14. Support (200) pour un article inhalateur (300) selon la revendication 13, dans lequel la première butée (216) peut être mise en prise avec le manchon (206) de telle sorte que la première butée (216) est déplacée de la position fermée à la position ouverte lorsque le manchon (206) est déplacé vers la position d'extraction.

15. Système inhalateur (100) comprenant un support (200) selon l'une quelconque des revendications précédentes et un article inhalateur (300).
